# FASCICULE DE BREVET EUROPEEN

(11) **EP 0 646 647 B1**
(45) Date de publication et mention de la délivrance du brevet: **15.01.2003**
(21) Numéro de dépôt: 94402237.5
(22) Date de dépôt: 05.10.1994
(51) Int. Cl.: C12P 1/00, C12P 39/00, C12N 1/20, C12N 1/12, A61K 35/02, A61K 7/48

(54) **Procédé de préparation de boues thermales, boues obtenues et leurs applications**
Verfahren zur Herstellung von Thermalschlamm, den hierdurch erhaltenen Schlamm und seine Anwendungen
Process for preparing thermal muds, muds thus obtained and their uses

(30) Priorité: 05.10.1993 FR 9311853
(43) Date de publication de la demande: 05.04.1995
(73) Titulaire: VILLE DE DAX, REGIE MUNICIPALE DES EAUX ET DE L'ASSAINISSEMENT, F-40100 Dax (FR)
(72) Inventeur: Counilh, Pascal, F-40300 Orist (FR); Gibert, Jean-Luc, F-40100 Dax (FR)
(74) Mandataire: Peaucelle, Chantal

(56) Documents cités:
- EP-A- 0 184 019
- CH-A- 286 680
- DE-C- 833 542
- FR-A- 2 474 865
- FR-A- 2 489 689
- FR-A- 2 558 480
- BIOFUTUR, no.76, Février 1989, PARIS FR pages 26 - 32 J.P. CALLEGARI 'L' EXPLOITATION DES MICROALGUES: ESSOR D'UNE TECHNOLOGIE.'

## Description

L'invention a pour objet un procédé de préparation de boues thermales. Elle se rapporte également aux nouvelles boues thermales élaborées et à leurs applications en particulier biologiques et biochimiques.

Il existe une vieille tradition d'utilisation des boues thermales dans toute l'Europe. La France y occupe une place importante avec de nombreuses stations proposant ce type de produit aux curistes. Ces boues thermales sont largement utilisées à titre curatif, en particulier en rhumatologie. On a ainsi recours à des applications localisées (illutations) ou à un enveloppement général (bain de boue). Les températures d'utilisation sont supérieures à 38°C, le plus souvent autour de 45°C et les durées de soin varient de 15 à 20 minutes.

Sous l'effet de la chaleur, le corps du malade subit des effets physiologiques importants tels qu' hypersudation, vasodilatation périphérique, stimulation de médiateurs chimiques. Il en résulte globalement une sensation de bien-être et une action sédative.

Selon le mode de préparation des boues thermales, elles peuvent en outre contenir des molécules organiques, capables de franchir la barrière cutanée, présentant un intérêt thérapeutique.

Une définition générale de l'expression "boue thermale" a été donnée par un groupe de travail réuni par le Ministère de la Santé en 1987, à Paris.

Selon cette définition, on entend par "boue thermale" des produits contrôlés issus d'une eau minérale (exemple : les précipités) ou du contact plus ou moins prolongé d'une eau minérale avec des matériaux résultant de processus géologiques (exemple : les argiles) ou géologiques et biologiques (exemple : les sédiments et les tourbes) et utilisés à des fins thérapeutiques dans la station d'origine de l'eau minérale".

D'une manière générale, ces boues sont à base d'un substrat argileux ou limoneux, imbibé d'eau minérale, et comportant une biocénose alguale et bactérienne.

On peut considérer qu'il existe deux grands groupes de boues différenciables par leur mode de préparation :
- les boues extemporanées pour lesquelles l'eau minérale est incorporée au substrat peu de temps avant leur utilisation. On peut constater que les échanges entre l'eau et le support sont exclusivement chimiques ;
- les boues maturées, ou péloïdes, où le temps de contact entre l'eau minérale et le substrat minéral est prépondérant et permet l'instauration d'échanges chimiques et biologiques.

On citera le péloïde de Dax qui a établi la réputation des établissements thermaux de la station de Dax qui l'exploitent.

A l'origine, on désignait, par l'appellation "Péloïde de Dax", le produit formé sur les bords de l'ADOUR, dans des cratères naturels où jaillissait l'eau thermale. Ces griffons étaient périodiquement submergés par les crues du fleuve et le limon se déposait, lors du retrait des eaux, dans ces cavités où venaient se plonger les malades pour profiter des bienfaits de la boue ainsi formée.

De nos jours, la maturation est l'opération qui permet de reproduire artificiellement les conditions naturelles qui ont été à l'origine de la production spontanée de ces boues. A DAX, elle consiste en un séjour prolongé, 6 mois et plus, dans des bassins où le contact entre l'eau chaude et le limon de l'ADOUR est favorisé. La température de l'eau minérale, supérieure à 55°C, inhibe la pousse de végétaux supérieurs et favorise le développement d'une flore, bioglée, spécifique, constituée d'un grand nombre d'algues et de bactéries particulières.

Des échanges entre les composants argileux et organiques (humus) du substrat et l'eau minérale interviennent. La maturation permet une concentration des éléments présents à l'état de trace et, par l'intermédiaire de la bioglée, elle apporte à la boue une série de molécules issues du métabolisme des algues et des bactéries, ainsi que des produits provenant de la dégradation de ces organismes. Le produit résultant de ces transformations biologiques et physico-chimiques est donc élaboré selon des processus naturels.

Les techniques de maturation utilisées jusqu'ici présentent cependant plusieurs inconvénients.

En effet, les caractéristiques du produit ne sont pas suffisamment reproductibles, notamment en ce qui concerne l'humidité, la densité, la minéralisation et la teneur en métabolites apparus au cours de la maturation. Ceci résulte en partie d'une mauvaise maîtrise des cycles bactériens et du faible rendement en algues.

De plus, le substrat organo-minéral, qui sert de base à la préparation du péloïde, est colonisé par de nombreux microorganismes difficiles à éliminer et dont certains peuvent présenter un caractère pathogène.

L'alimentation en permanence des bassins de préparation en eau entraîne une consommation très importante d'eau thermale de l'ordre de 350 m³ d'eau par m³ de péloïde préparé.

Il s'agit en outre de procédés qui ne sont pas adaptés à la livraison de petites quantités de produit en basse saison, dont les conditions d'exploitation manquent de souplesse, et qui ne sont pas adaptés à l'exploitation de nouvelles sources de substrat lorsque les gîtes traditionnels seront épuisés.

Le mode de livraison actuel entraîne, par voie naturelle, une dessication du produit qui altère sa qualité et rend les lots livrés très hétérogènes.

Le brevet DE-A-833 542 décrit un procédé de fabrication d'une pâte pour bain au cataplasme qui comprend la préparation d'une part d'une substance d'ensemencement comme la tourbe blanche ou blonde, à laquelle on ajoute de l'eau et des bactéries dont la croissance provoque un auto-échauffement, d'autre part d'une pâte de base, pouvant être également constituée par de la tourbe blanche ou blonde, à laquelle sont incorporés des hydrates de carbone aux fins de fermentation et des composés minéraux, la substance d'ensemencement et la pâte de base étant mélangées.

Le brevet CH-A-286 680 concerne la préparation d'agents thérapeutiquement efficaces à partir de péloldes. Une bouillie de péloides est alors élaborée dans laquelle s'effectue diverses réactions.

Le brevet FR-A-2 558 480 concerne la préparation de boues marines enrichies en algues pour utilisation en balnéothérapie, cosmétologie, ou sous la forme de spécialités pharmaceutiques.

Pour disposer de boues thermales satisfaisantes et remédier aux problèmes évoqués ci-dessus, les inventeurs ont mis au point des procédés de culture respectivement d'algues et de bactéries permettant de disposer avec une grande maîtrise de ces produits en grande quantité pour élaborer des boues thermales répondant aux caractéristiques requises dans différentes applications.

Ils ont également mis au point un procédé permettant de contrôler sa préparation mécanique et son conditionnement.

L'invention a donc pour but de fournir un nouveau procédé de préparation de boues thermales dans des conditions contrôlées, de grande souplesse d'utilisation, permettant notamment d'améliorer la reproductibilité des caractéristiques essentielles de ces boues, de réduire la consommation d'eau thermale, et d'éliminer les éventuelles contaminations bactériologiques.

Elle vise également à fournir une chaîne de préparation permettant de réaliser l'ensemble des étapes opératoires pour l'obtention de ces boues thermales, y inclus les cultures des bactéries et des algues, en optimisant les conditions de préparation.

Elle vise également à fournir, en tant que produits nouveaux, les boues thermales ainsi élaborées.

Selon encore un autre aspect, l'invention vise les applications biochimiques et biologiques de ces boues thermales aussi bien pour l'homme, en particulier en thermalisme et en cosmétologie, que pour l'animal.

Le procédé de préparation de boues thermales selon l'invention est caractérisé en ce qu'il comprend :
- le mélange d'une pâte fluide, élaborée en ajoutant de l'eau minérale à un substrat organo-minéral constitué par un matériau argileux ou limoneux comprenant une biocénose alguale et bactérienne, avec des bactéries thermophiles du cycle du soufre,
- la maturation, sous agitation, à une température appropriée, de manière à activer la formation de métabolites par les bactéries et les algues éventuellement ajoutées et l'imprégnation du substrat,
- la récupération du mélange et son traitement.

Par l'expression "eau minérale", on entend, dans la description et les revendications, aussi bien des eaux naturellement minéralisées, provenant des sources naturelles d'eaux minérales contenant des ions thérapeutiquement actifs, ou des eaux enrichies en minéraux donnés contenant de tels ions.

Ces eaux minérales, en particulier celles provenant de sources naturelles, renferment des flores alguales et bactériennes adaptées à la température de l'eau, et généralement des constituants résultant de leur dégradation et les produits issus de leur métabolisme.

Selon le mode de réalisation le plus général de l'invention, le substrat organo-minéral est un matériau argileux tel que résultant de processus biologiques, ou géologiques et biologiques. Il s'agit avantageusement d'un matériau présentant un comportement thermique et une capacité d'échange satisfaisants.

Un tel matériau est maintenu, après extraction, en contact avec de l'eau, pendant la durée de son stockage pour éviter toute dessication du produit et conserver son pouvoir réducteur lorsque le substrat possède initialement des propriétés réductrices.

La pâte fluide est élaborée en imbibant le substrat organo-minéral avec une eau minérale. Le temps de contact entre le substrat et l'eau minérale doit assurer des échanges satisfaisants, c'est-à-dire jusqu'à saturation du complexe absorbant.

Conformément à l'invention, on mélange à la pâte fluide des bactéries, et le cas échéant des algues.

Les termes "algues" et "bactéries" désignent, selon l'invention aussi bien les produits en tant que tels que les ensemencements d'algues et les inoculums bactériens qui renferment également les constituants provenant de la dégradation des algues et des bactéries et les produits de leur métabolisme.

Il s'agit d'algues et de bactéries thermophiles c'est-à-dire capables de se développer à des températures supérieures à l'ambiante, et même de 45°C ou plus, voire de 56 à 60°C.

Les algues ajoutées à la pâte fluide sont des algues d'eau douce ou des algues sélectionnées par la température spécifique des eaux minérales.

Des familles d'algues de ce dernier type comprennent les Cyanophycées ou Cyanobactéries, et/ou les Chlorophycées et/ou les Diatomées.

Parmi les Cyanophycées, on citera l'ordre Chroococcales avec notamment Chroococcus minutus et Synechococcus elongatus, ou encore l'ordre Oscillatoriales avec, notamment, Oscillatoria formosa, Oscillatoria germinata, Oscillatoria granulata, Phormidium ambigum, Phormidium autumnale, Phormidium corium, Phormidium luridum, Phormidium tenue, Phormidium uncinatum, Phormidium valderianum, Anabaena sp., Anabaena constricta, Anabaena variabilis, Noctoc sp., Cylindrospermum stagnale, Mastigocladus laminosus, Mastigocladus laminosus forma anabaenoides, Stigonema sp., Calothrix thermalis. Les Chlorophycées sont en particulier de l'ordre Volvocales, comme Chlamydomonas sp., ou Ulothricales, comme Hormidium crassum, ou encore de l'ordre Chloroccales, comme Scenedesmus quadricauda, Scenedesmus acutiformis, Scenedesmus obliquus, Ankistrodesmus falcatus ou Micractinium pusillum.

Parmi d'autres algues appropriées pour la mise en oeuvre de l'invention, on citera les Diatomées, par exemple celles de l'ordre Centrales, comme Cyclotella sp. ou Nelosira varians, ou de l'ordre Pennales, comme Nitzschia palea, Nitzschia thermalis, Pinnularia interrupta forma minutissima, Hantzschia amphioxys, Amphora holsatica.

Ces algues sont ajoutées à la pâte fluide à raison de 0,02 à 0,15 % en poids sec et, de préférence, de 0,05 à 0,10 %, notamment de l'ordre de 0,08 %.

On observera à cet égard l'enrichissement considérable du substrat en algues, alors que la teneur habituelle en algues des produits obtenus par un procédé naturel est inférieure à 0,01 %.

Des valeurs supérieures peuvent être naturellement utilisées en effectuant les cultures d'algues à plus grande échelle que celle rapportée dans ces exemples.

Ces algues sont utilisées avantageusement sous forme d'un broyat. Ce broyat est par exemple préparé à partir d'algues conservées à l'état congelé, soumises à une décongélation selon les besoins et traitées pour obtenir une solution homogénéisée.

Selon une disposition avantageuse de l'invention, les algues sont cultivées selon un procédé comportant le ruissellement en continu sous lumière naturelle et/ou artificielle d'une eau constituant un milieu nutritif de culture, à une température appropriée pour le développement de l'algue, sur un support de culture en plan incliné, et la récupération des algues.

Les souches d'algues sont ensemencées sur ce support ou sont apportées par l'eau utilisée qui est avantageusement une eau minérale et plus spécialement une eau hyperthermale c'est-à-dire une eau minérale dont la température est supérieure à environ 45°C.

Dans ce cas, l'eau apporte également la température requise pour le développement des algues, cette température étant d'au moins 45°C.

L'eau utilisée est éventuellement supplémentée en nutriments, par exemple azotés ou phosphorés.

Le débit de circulation de l'eau minérale, l'inclinaison du plan de culture et la température sont choisis selon les contraintes de la culture.

A titre d'exemple, avec une eau minérale à une température de l'ordre de 50 à 56°C, on effectue avantageusement le ruissellement avec un débit d'eau par mètre linéaire de culture de 0,1 à 0,5 m³/h, sur un plan incliné d'environ 15 à 20°.

Pour réduire la baisse de température dans la partie basse de la culture, on utilise un support de culture avantageusement chauffé, par exemple, par un serpentin dans lequel circule l'eau minérale chaude et/ou un câble chauffant.

La surface du support de culture est choisie, notamment en ce qui concerne le matériau et la rugosité de manière à permettre un accrochage satisfaisant des algues. On utilise par exemple avantageusement, une surface rainurée.

Selon une variante de réalisation du procédé de culture, on opère en circuit ouvert, l'eau minérale étant récupérée, après ruissellement, pour d'autres applications.

Une autre variante est réalisée en circuit fermé, l'eau minérale étant récupérée au pied de la culture, réchauffée à la température souhaitée et remontée en tête de culture.

Les algues sont collectées par raclage de la surface de la culture, égouttées, puis essorées par centrifugation. A des fins de conservation, ces algues sont congelées à basse température, puis, en fonction des besoins, un ensemencement est préparé aux fins d'addition à la pâte fluide comme indiqué plus haut. Comme déjà indiqué, cet ensemencement comprend, outre les algues, leurs constituants et leurs métabolites.

Ce procédé permet d'obtenir à grande échelle des algues utilisables pour l'élaboration de boues thermales, et notamment d'algues spécifiques des eaux hyperthermales, comme les eaux minérales de Dax.

Les bactéries utilisées dans le procédé de l'invention, sont plus spécialement des bactéries du cycle du soufre, telles que les Thiobacillus ou celles du genre Clostridium bifermentans. On utilise avantageusement celles capables de sécréter des polymères, de manière à augmenter l'onctuosité de la boue. Dans le cas où le procédé comporte la mise en oeuvre d'eaux minérales sulfatées, on a recours à des bactéries capables notamment d'activer la production de composés réduits du soufre à partir des sulfates ou des sulfites de l'eau minérale .

Des bactéries présentant à la fois ces propriétés de sécrétion de polymères et cet effet sulfato ou sulfito-réducteur sont avantageusement telles qu'obtenues à partir de cultures, développées en fermenteur, de Clostridium bifermentans.

Ces cultures sont avantageusement réalisées à une température de l'ordre de 38°C, sous agitation, avec contrôle du pH pour un ajustement à 6,0 ± 0,2.

Des conditions de production économiquement intéressantes sont obtenues avec un ensemencement de l'ordre de 106 bactéries/gramme de pâte. Cette valeur correspond déjà à un enrichissement considérable du substrat en bactéries étant donné qu'une boue thermale naturelle, par exemple, ne comporte qu'environ 10⁴ bactéries de ce type par gramme de pâte.

On dispose ainsi d'un matériau fortement enrichi, de manière contrôlée, en algues et/ou en bactéries et en leurs constituants et métabolites.

Pour augmenter la teneur du matériau en dérivés soufrés, on ajoute en outre au mélange des sulfates et/ou des sulfites ou autres dérivés soufrés. Des doses appropriées sont de l'ordre de 1 à 3g/kg de pâte.

Selon une disposition supplémentaire de l'invention, le mélange ci-dessus est soumis à une étape de maturation à une température appropriée, avantageusement sous agitation, de manière à activer la formation de métabolites par les algues et/ou les bactéries et l'imprégnation du substrat.

La durée du traitement de maturation est d'environ au moins 30 jours.

L'étape de maturation peut être réalisée en aérobiose, dans un réacteur ouvert, lorsqu'on utilise une pâte oxydée.

Le plus généralement, compte-tenu des propriétés intéressantes des dérivés réduits du soufre, on opère en anaérobiose, dans un réacteur fermé, sous agitation.

Lorsque cette étape de maturation est réalisée en présence de bactéries, cette phase permet la transformation du substrat sous l'effet des cycles bactériens qui vont se développer et utiliser la matière organique initiale du substrat et/ou celle provenant des algues ajoutées. Les produits qui se forment imbibent le substrat.

Comme rappelé à propos des algues, l'ensemencement comprend les bactéries, leurs constituants et leurs métabolites.

Avec un inoculum de C. bifermentans, la maturation est réalisée à une température d'au moins 35°C environ, notamment de l'ordre de 38°C.

Les composés réduits de soufre qui se forment confèrent à la boue une couleur noirâtre et une odeur caractéristique.

La boue formée, appelée boue thermale compte tenu de son intérêt en thermalisme, est récupérée en vue de son conditionnement.

L'eau d'imbibition est au moins en partie évacuée de manière à obtenir une boue présentant la viscosité requise par les applications envisagées, ayant plus spécialement une consistance pâteuse.

Cette eau est riche en métabolites et produits de la maturation et peut donc être récupérée aux fins d'autres applications.

La dessiccation partielle du produit peut être réalisée par différents systèmes, par exemple par des procédés physiques et/ou par addition de produits secs.

Les procédés physiques comprennent notamment le chauffage du produit dans une enceinte maintenue sous-vide, l'utilisation de filtres-presses, de tamis vibrants ou de décanteurs centrifuges.

Les produits secs ajoutés sont avantageusement essentiellement formés soit par de la boue préparée, séchée et broyée, soit par de la boue lyophilisée et pulvérisée. Cette pratique, à partir de la boue elle-même, permet de garder au produit 100 % de l'argile d'origine. Pour réaliser cette opération, les doses de boue sèche sont d'environ 300 à 700 grammes par kg de matière à déshydrater. On peut également incorporer des argiles en poudre provenant de l'industrie. Avec des argiles de surface spécifique plus importante et donc plus réactive, on peut diminuer les doses entre 150 et 300 g/kg.

Une autre solution avantageuse consiste à ajouter, le cas échéant en plus des produits ci-dessus, des diatomées séchées et broyées qui sont présentes naturellement dans la boue. Ainsi, les doses sont de 40 à 80 grammes de diatomées par kg de boue à déshydrater.

En variante, on ajoute des produits organiques, le cas échéant en plus des produits mentionnés ci-dessus. Certains de ces produits à base d'hydroxyméthylcellulose ont une bonne capacité d'absorption de l'eau. Les doses optimales sont voisines de l'ordre de 0,05 à 0,01 %. Il convient de s'assurer que ces produits sont conformes aux spécifications de la Pharmacopée.

Le conditionnement peut être alors automatisé pour effectuer une livraison extérieure au lieu de préparation.

Dans un mode préféré de mise en oeuvre de l'invention, on établit une chaîne pour la préparation de boues thermales en juxtaposant les opérations élémentaires de culture d'algues, de culture de bactéries, de préparation du substrat, et de mélange de la pâte fluide ou, le cas échéant, de maturation.

Cette juxtaposition est rendue possible grâce à la réalisation de cultures d'algues à grande échelle, selon le procédé de l'invention, en particulier d'algues spécifiques d'eaux hyperthermales, ainsi qu'à l'obtention de souches bactériennes, à leur culture et à leur ensemencement comme indiqué plus haut.

D'une manière générale, le procédé de l'invention permet le contrôle des différentes matières premières utilisées à chacune des étapes de la chaîne de préparation ainsi que celui des produits obtenus, assurant ainsi l'obtention des qualités requises pour le produit final.

L'invention vise également en tant que nouveaux produits les boues ainsi élaborées.

Ces boues sont du type des boues thermales contenant un substrat organo-minéral imbibé d'une eau minérale comportant une biocénose alguale et bactérienne ainsi que des ions à effet thérapeutique et sont caractérisées en ce qu'elles se présentent sous forme d'une composition homogène de matériau argileux ou limoneux, à l'exclusion de boues marines, enrichie en bactéries thermophiles du cycle du soufre et le cas échéant en algues d'eau douce ou en algues spécifiques des eaux thermales, à raison d'un facteur d'au moins environ 10 pour les algues et 10² pour les bactéries par rapport au substrat organo-minéral de départ traité, et le cas échéant enrichie également en dérivés soufrés réduits.

Les algues et les bactéries sont telles que définies ci-dessus.

Ces boues comportent en outre la microflore bactérienne et les produits du métabolisme de cette microflore provenant du substrat et de son eau d'imbibition, des algues et de leur culture, de nombreuses bactéries étant associées à la prolifération des algues et des bactéries résultant de l'inoculum ajouté pour leur préparation.

Elles peuvent donc renfermer des bactéries dont l'habitat est l'eau et le sol et les produits de leur métabolisme.

Ces bactéries appartiennent à tous les cycles physiologiques, de l'azote, du carbone, du phosphore, du soufre, du fer et autres.

Un groupe tout particulièrement préféré correspond à des boues enrichies en algues et en bactéries, et le cas échéant en produits soufrés réduits.

Des boues de ce groupe sont plus spécialement telles qu'obtenues selon le procédé de maturation défini ci-dessus.

Grâce à la reproductibilité du procédé de l'invention et à l'obtention à grande échelle des algues et des bactéries, l'invention permet de disposer de produits de grande fiabilité pour de nombreuses applications.

Les propriétés de ces boues enrichies en bactéries, et le cas échéant en algues, sont mises à profit dans les applications classiques en thermalisme.

En raison de leur enrichissement également en de nombreux métabolites actifs, on constate des résultats nettement améliorés lors de leur utilisation pendant des cures. Ces effets bénéfiques sont observés sur l'homme ou l'animal, par exemple le cheval.

Ces boues sont également utilisables dans des applications paramédicales.

L'invention vise donc les préparations à usage paramédical renfermant ces boues.

Ces préparations sont caractérisées en ce qu'elles renferment une quantité efficace de boues de l'invention associées à un véhicule facilitant son application.

Pour effectuer par exemple des massages, on les mélange à une base de paraffine, ou encore à de la méthylcellulose ou de la lanovaseline.

Selon un autre aspect, les boues de l'invention présentent également un grand intérêt en cosmétologie.

Les essais réalisés ont en effet montré une action avantageuse sur la peau après application de différentes formulations renfermant ces boues.

L'invention vise donc également les préparations cosmétologiques renfermant une quantité efficace des boues évoquées plus haut, associées à un véhicule inerte approprié.

Comme véhicule approprié pour l'élaboration de ces formulations, on citera notamment des émulsifiants, des alginates ou de la méthylcellulose.

Ces formulations, selon une variante, sont enrichies en algues thermophiles.

D'autres caractéristiques et avantages de l'invention sont donnés dans les exemples qui suivent.

### Exemple 1 : Procédé de préparation de boues thermales maturées de type péloïdes.

### 1 extraction du limon argileux fossile.

Le limon argileux de l'Adour utilisé se présente sous la forme d'un matériau argileux compact, dont les caractéristiques sont les suivantes :
densité : 1,9
humidité : 30 %
classification granulométrique :
   (45 % argile, 45 % limon, 10 % sable))
texture : argilo-limoneuse
couleur : gris-bleu
matières organiques : de 3 à 10 %
minéralogie des argiles :
Quartz P/F
Plagioclase infra Tr
Anhydrite (probable) infra Tr

### Argiles A/P

Smectite environ 2,5 (Ro à forte proportion d'illite [ [environ 80 %] )
Illite 3
Interstratifié Illite - chlorite Tr
Chlorite 1,5
Kaolinite mal cristallisée ou métahalloysite 3
(les valeurs sont en % en poids).

Le comportement thermique et les échanges cationiques et anioniques obtenus avec ce matériau sont particulièrement satisfaisants.

L'extraction est réalisée par strates successives après décapage de la couche de terre végétale, des alluvions sablonneuses et de tout matériau impropre au procédé.

### 2 Stockage sous eau.

Le limon prélevé sous forme de mottes est déposé dans des bassins de stockage et recouvert d'une couche d'eau.

L'addition d'eau permet d'éviter la dessication du produit et facilite sa désagrégation en petites mottes, ce qui rend ensuite plus aisé le travail mécanique du limon argileux.

On utilise une capacité de stockage de 6 000 m³, divisée en 40 bassins de 150 m³.

Le stockage, effectué à température ambiante, peut durer de nombreux mois, selon les prévisions de fréquentation, mais ne doit pas être inférieur à une année.

### 3 Préparation d'une pâte fluide.

Le limon argileux stocké sous eau est extrait selon les besoins en alimentation de la chaîne de préparation des boues. On forme une pâte fluide onctueuse imbibée d'eau minérale en délayant le limon argileux dans une cuve d'eau minérale sulfatée calcique à une température de 56°C.

La mesure de la balance ionique de l'eau d'imbibition résultant du contact entre l'eau minérale et le substrat arigileux est la suivante :
- cations, en mg/l : Na⁺ 940,00 ; K⁺ 15,00 ; NH₄⁺ 0,819 ; Ca⁺⁺ 13000 ; Mg⁺⁺ 4,016 ; Fe⁺⁺ 0,000, ce qui correspond à un total de 1278,55 mg/l ;
- anions en mg/l : CO₃⁻⁻ 0,00 ; HCO₃⁻ 250,95, SO₄⁻⁻ 2430,00 ; Cl⁻177,00 ; NO₂⁻ 0,00 ; NO₃⁻ 1,87 ; PO₄⁻⁻ 0,00, ce qui correspond à un total de 2859,82 mg/l.

L'humidité est amenée à 50 % environ. On laisse le produit se déliter pendant environ 2 heures afin d'obtenir des échanges optima entre l'eau et le limon argileux.

La pâte fluide résultante est avantageusement tamisée à 500 µ, introduite dans le réacteur, puis on ajoute progressivement les algues, et leurs métabolites à raison de 0,08 % en poids sec et un inoculum bactérien de 106 colonies de C. bifermentans/g de pâte

On ajoute également, à titre de complément, 2g de sulfates/kg de pâte.

Les caractéristiques du mélange ainsi formé à l'entrée du réacteur sont les suivantes :
- densité :: 1,35 - 1,55
- humidité :: 49 - 51 %
- viscosité :: 16000 - 19000 CP
- pH :: 6,7 ± 0,2
- potentiel d'oxydo-réduction :: < - 150 mV
CEC (capacité d'échange ou capacité d'échange cationique):taux de saturation 73 %
Clostridium bifermentans ensemencé à raison de 106 colonies par gramme
algues thermales inoculées à 0,08 % en poids sec.

### 4. Maturation en réacteur clos.

La maturation est réalisée en anaérobie dans des réacteurs clos de 10 à 20 m³, à une température minimale de 38°C. Le milieu est soumis à agitation à 50 tpm. Le temps de séjour dans le réacteur est d'au moins 1 mois.

Les réacteurs utilisés se présentent sous forme de cuves circulaires en béton, isolées par l'extérieur, munies sur leur paroi intérieure d'un serpentin où circule de l'eau minérale chaude. Une sonde de température placée au centre de la cuve commande par l'intermédiaire d'un boîtier de contrôle programmé à une température de consigne, une électro-vanne, qui met en circulation le fluide chaud à 56°C.

A l'aide de la chaleur, les cycles bactériens vont se développer, utilisant la matière organique initiale du produit ou celle provenant des algues ajoutées, et permettre l'obtention de produits nouveaux imbibant la boue et l'apparition d'une couleur noirâtre, due aux composés réduits du soufre.

Les caractéristiques du produit fini sont les suivantes :
- densité :: 1,45 à 1,55
- humidité :: de 49,0 à 51,0 %
- couleur :: gris-noir

### 5. Conditionnement du produit maturé.

Le produit maturé est récupéré au bas des réacteurs.

Une partie de l'eau d'imbibition du produit est évacuée pour obtenir une pâte épaisse, de viscosité compatible avec les applications envisagées, par exemple l'étalement sur la peau du curiste.

Aux fins de pasteurisation, la boue récupérée est soumise à un traitement thermique, puis expédiée sur un poste de conditionnement, qui permet le remplissage automatique de récipients et leur sertissage. Ces récipients sont avantageusement munis d'un sac plastique en un matériau inerte, qui rend l'emballage étanche et évite la dessication de la boue thermale et son oxydation. La boue ainsi traitée est stockée en attendant la livraison, sans perdre ses qualités, grâce à l'étanchéité obtenue vis-à-vis de l'air et de la lumière.

Les caractéristiques du péloïde sont vérifiées, en particulier les paramètres suivants : densité, humidité, viscosité, pH, redox, matières organiques, granulométrie, comportement thermique, eau d'imbibition, chimie, CEC, microbiologie, biochimie (sucres, acides aminés), et si besoin minéralogie.

La composition en sulfures est la suivante : sulfures en ions S déterminés par colorimétrie après extraction à l'eau acide, 190 mg/kg. La teneur en sucres totaux est inférieure à 150 mg/kg.

La composition en acides aminés libres, telle qu'obtenue au cours de différentes expériences, est donnée ci-après :

| | |
|---|---|
| TAURINE | < 5 mg/kg |
| ACIDE ASPARTIQUE | 70 - 80 mg/kg |
| THREONINE | 35 - 60 mg/kg |
| SERINE | 40 - 50 mg/kg |
| ACIDE GLUTAMIQUE | 5 - 50 mg/kg |
| PROLINE | 5 à 75 mg/kg |
| GLYCINE | 5 - 35 mg/kg |
| ALANINE | 35 - 95 mg/kg |
| CYSTINE | 100 - 150 mg/kg |
| VALINE | 30 - 50 mg/kg |
| METHIONINE | < 5 mg/kg |
| ISOLEUCINE | 10 - 25 mg/kg |
| LEUCINE | 15 - 35 mg/kg |
| TYROSINE | 5 - 55 mg/kg |
| PHENYLALANINE | 5 - 15 mg/kg |
| HISTIDINE | 5 - 35 mg/kg |
| LYSINE | 35 - 80 mg/kg |
| ARGININE | 5 - 25 mg/kg |

On contrôle également le résultat de la pasteurisation qui doit montrer l'absence de C. albicans, de P. aeruginosa, de S. aureus, de coliformes thermotolérants < 10². Les souches de Clostridium peuvent être retrouvées à raison de 10² à 10⁶ bactéries/g de boue, selon les prélèvements.

### Exemple 2 : Procédé de culture d'algues spécifiques d'eaux thermales.

On soumet la culture réalisée à un ruissellement permanent d'eau minérale chaude, sur un support incliné, en opérant à la lumière naturelle et/ou artificielle. Le cycle entre deux récoltes est de 30 jours. Cette eau minérale sert de milieu nutritif et véhicule les souches d'algues que l'on souhaite cultiver. Elle est enrichie en nutriments azotés et phosphorés. Le rendement moyen obtenu est de 1400 mg de poids sec d'algues par décimètre carré pour un cycle de culture. Pour protéger les surfaces de culture, les rampes sont couvertes par une serre horticole, équipée d'un film translucide de 200 µ, laissant passer 98 % de luminosité.

L'inclinaison du support est de 18°, le débit de circulation de l'eau thermale est de 0,3 m³/h par mètre linéaire de culture. Le gradient de température de la culture est de 56°C à 45°C entre le haut et le bas de l'écoulement. Pour limiter la baisse trop rapide de la température, le support de la culture est réchauffé par un serpentin d'eau minérale et/ou un câble chauffant.

Le profil de la surface est rugueux.

En fonction des conditions saisonnières, deux modes de mise en oeuvre sont adoptés :
- le premier est réalisé en circuit ouvert : l'eau minérale après ruissellement est récupérée par une goulotte et réservée à d'autres applications.
- le second est réalisé en circuit fermé, l'eau thermale est récupérée au pied de la culture, réchauffée à 56°C et remontée en tête de culture. Le système de réchauffage est asservi à une mesure de température sur l'eau au bas de la pente, avec un point de consigne de 45°C.

En fin de cycle, les algues sont collectées par raclage de la surface de culture. Après égouttage et essorage par centrifugation, les algues sont congelées à -20°C.

En fonction des besoins, un ensemencement d'algues est préparé selon la teneur requise pour être ajouté à la boue au début de la maturation. Les algues sont décongelées. Une partie est hachée par homogénéiseur type ultra-turrax, un sonificateur à flux continu permet le traitement de l'autre partie. Les solutions obtenues sont à nouveau homogénéisées pour être introduites dans le réacteur et réparties par portions.

### Exemple 3 : Préparation d'un inoculum bactérien de C. bifermentans.

On rapporte ci-après la culture de **C. bifermentans** en fermenteur. Il s'agit de la souche déposée à l'ATCC sous le n° 19299.

Le milieu de culture utilisé présente la composition suivante :

| | Composants |
|---|---|
| - glucose | 40 |
| - extrait de levure | 10 |
| - peptone | 10 |
| - tryptone | 10 |
| - amidon soluble | 1 |
| - cystéine | 0.5 |
| - chlorure de sodium | 5 |
| - acétate de sodium | 3 |
| - agar-agar | 0.5 |
| - sulfate d'ammonium | 3 |
| - hydrogénophosphate de potassium | 3 |

Deux systèmes de culture ont été retenus :
- un système en "batch",
- un système avec recyclage de la biomasse.

Dans les deux cas, la température de la culture est de 38°C, la régulation du pH se fait par ajout d'ammoniaque à 14 ou 28 % et l'agitation varie de 200 à 300 tpm.

### Exemple 4 : utilisation des boues de l'invention en thermalisme.

Les boues maturées obtenues selon l'exemple 1 sont utilisées sous forme d'applications localisées à une température variant de 38 à 45°C, durant 15 à 20 min., ou de bains complets à une température supérieure à 40°C durant 10 à 20 min. Le traitement est appliqué 1 fois/jour, pendant 3 semaines.

### Exemple 5 : utilisation des boues de l'invention en cosmétologie.

- **Formulation d'une pâte de gel**
   On mélange les ingrédients suivants :
   boue de l'invention : 30 %
   méthylcellulose : 3 %
   eau minérale : qsp 100
- **formulation pour base de crème**
   on mélange les ingrédients suivants :
   boue de l'invention : 5 à 30 %
   lanovaseline qsp : 100
   eau minérale : 10 ml
- **formulation pour bain clair.**

A 4 à 10 % de boue de l'invention, on ajoute de l'eau minérale à raison de 96 à 90 %. Le bain clair peut être enrichi en algues, telles qu'obtenues selon le procédé de culture d'algues de l'invention, en utilisant un ensemencement de 0,01 à 0,1 % en poids sec d'algues par rapport au poids du bain.

## Revendications

1. Procédé de préparation de boues thermales, **caractérisé en ce qu'**il comprend les étapes de
- mélange d'une pâte fluide, élaborée en ajoutant de l'eau minérale à un substrat organo-minéral constitué par un matériau argileux ou limoneux comportant une biocénose alguale et bactérienne, avec des bactéries thermophiles du cycle du soufre, et le cas échéant des algues d'eau douce ou des algues spécifiques des eaux hyperthermales,
- maturation, sous agitation, à une température appropriée, de manière à activer la formation de métabolites par les bactéries, et les algues lorsqu'elles sont utilisées, et imprégnation du substrat,
- récupération du mélange et son traitement.

2. Procédé selon la revendication 1, **caractérisé en ce qu'**on utilise des Cyanophycées ou Cyanobactéries et/ou des Chlorophycées et/ou des Diatomées.

3. Procédé selon la revendication 2, **caractérisé en ce que** les algues sont ajoutées à la pâte fluide à raison de 0,02 à 0,15 % en poids sec.

4. Procédé selon la revendication 3, **caractérisé en ce que** les algues sont ajoutées à raison de 0,05 à 0,10 % en poids sec.

5. Procédé selon l'une des revendications 1 à 4, **caractérisé en ce que** les bactéries ajoutées à 1a pâte fluide sont choisies parmi les bactéries capables d'activer la production de composés réduits du soufre à partir des sulfates de l'eau minérale et/ou de sécréter des polymères à caractère onctueux.

6. Procédé selon la revendication 5, **caractérisé en ce qu'**on utilise des bactéries du genre Clostridium bifermentans

7. Procédé selon la revendication 6, **caractérisé en ce que** les bactéries sont ajoutées dans le réacteur à raison de 10⁶ bactéries/g de pâte.

8. Procédé selon l'une quelconque des revendications 1 à 7, **caractérisé en ce qu'**on ajoute des sulfates et/ou des sulfites et autres dérivés soufrés, à raison de 1 à 3g/kg de pâte.

9. Procédé selon l'une des revendications précédentes, **caractérisé en ce que** l'étape de maturation est réalisée en aérobiose, dans un réacteur ouvert lorsqu'on utilise des pâtes oxydées.

10. Procédé selon l'une des revendications 1 à 8, **caractérisé en ce que** l'étape de maturation est réalisée en anaérobiose, dans un réacteur fermé, lorsqu'on utilise des pâtes à caractère réducteur.

11. Procédé selon la revendication 10, **caractérisé en ce que** la maturation est réalisée à une température supérieure de 35° à 38°C, lorsqu'on utilise un inoculum de C.bifermentans, pendant au moins 30 jours.

12. Procédé selon l'une des revendications 1 à 11, **caractérisé en ce qu'**on évacue une partie de l'eau d'imbibition de la boue récupérée, puis on soumet la boue à un traitement thermique aux fins de pasteurisation, et aux opérations de conditionnement.

13. Procédé selon l'une des revendications précédentes, **caractérisé en ce qu'**il comprend l'addition d'algues obtenues par ruissellement en continu sous lumière naturelle et/ou artificielle d'une eau constituant un milieu nutritif de culture, à une température appropriée pour le développement de l'algue, sur un support de culture en plan incliné.

14. Procédé selon la revendication 13, **caractérisé en ce que** les souches d'algues sont ensemencées sur ce support ou sont apportées par l'eau hyperthermale utilisée, c'est-à-dire une eau minérale dont la température est supérieure à environ 45°C.

15. Procédé selon la revendication 14, **caractérisé en ce que** l'eau est supplémentée en nutriments, azotés ou phosphorés.

16. Procédé selon la revendication 14 ou 15, **caractérisé en ce qu'**avec une eau minérale à une température de 50 à 56°C, on effectue le ruissellement avec un débit d'eau, par mètre linéaire de culture, de 0,1 à 0,5 m³/h sur un plan incliné de 15 à 20°.

17. Procédé selon l'une des revendications 13 à 16, **caractérisé en ce que** le support de culture est chauffé afin de réduire la baisse de température dans la partie basse du plan incliné.

18. Procédé selon la revendication 5, **caractérisé en ce que** les bactéries ajoutées sont cultivées en fermenteur, à une température de 38°C, sous agitation, avec contrôle du pH à une valeur de 6,0 ± 0,2.

19. Procédé de préparation de boues thermales, **caractérisé en ce qu'**il comprend dans une même chaîne de préparation les étapes définies dans les revendications 1 à 18.

20. Boues du type des boues thermales, contenant un substrat organo-minéral imbibé d'une eau minérale comportant une biocénose alguale et bactérienne ainsi que des ions à effet thérapeutique, **caractérisées en ce qu'**elles se présentent sous forme d'une composition homogène de matériau argileux ou limoneux, et comportent au moins 10⁶ bactéries thermophiles du cycle du soufre/g.

21. Boues selon la revendication 20, **caractérisées en ce qu'**elles renferment des dérivés soufrés réduits.

22. Boues selon l'une quelconque des revendications 20 ou 21, **caractérisées en ce qu'**elles sont additionnées d'algues d'eau douce ou d'algues spécifiques des eaux thermales à raison de 0,1% en poids sec.

23. Application des boues selon l'une quelconque des revendications 20 à 22, ou telles qu'obtenues par le procédé selon l'une quelconque des revendications 1 à 19 en thermalisme.

24. Préparation à usage paramédical, **caractérisée en ce qu'**elle renferme des boues selon l'une quelconque des revendications 20 à 22, ou telles qu'obtenues par le procédé selon l'une des revendications 1 à 19, associées à un véhicule facilitant leur application, du type de la paraffine, de la lanovaseline ou de la méthylcellulose.

25. Préparation cosmétologique, **caractérisée en ce qu'**elle renferme une quantité efficace de boues selon l'une quelconque des revendications 20 à 22, ou telles qu'obtenues par le procédé selon l'une des revendications 1 à 19, en association avec un véhicule inerte approprié pour les applications en cosmétologie.

26. Préparation cosmétologique selon la revendication 25, **caractérisée en ce qu'**elle renferme des algues thermophiles telles que définies dans la revendication 3.

27. Application vétérinaire des boues selon l'une quelconque des revendications 20 à 22, ou telles qu'obtenues par le procédé selon l'une des revendications 1 à 19.

## Patentansprüche

1. Verfahren zum Zubereiten von Thermalschlämmen, **dadurch gekennzeichnet, dass** es die Schritte umfasst :
- Mischung einer flüssigen Paste, hergestellt durch Hinzufügen von Mineralwasser zu einem organo-mineralischen, durch ein toniges oder schlammiges Material gebildeten Substrat mit einer algen- und bakterienhaltigen Biozönose, mit thermophilen Bakterien des Schwefelzyklus und ggf. mit Süßwasseralgen oder spezifischen Heißwasser-Algen,
- Reifen unter Rühren bei einer zum Anregen der Bildung von Metaboliten durch die Bakterien, und ggf. durch die Algen, wenn sie verwendet werden, geeigneten Temperatur und Tränken des Substrats,
- Rückgewinnung der Mischung und ihre Verarbeitung.

2. Verfahren nach Anspruch 1, **dadurch gekennzeichnet, dass** man Cyanophyceen oder Cyanobakterien und/oder Chlorophyceen und/oder Diatomeen verwendet.

3. Verfahren nach Anspruch 2, **dadurch gekennzeichnet, dass** die Algen der flüssigen Paste zu 0,02 bis 0,15 % des Trockengewichts zugesetzt werden.

4. Verfahren nach Anspruch 3, **dadurch gekennzeichnet, dass** die Algen zu 0,05 bis 0,10 % des Trockengewichts zugesetzt werden.

5. Verfahren nach einem der Ansprüche 1 bis 4, **dadurch gekennzeichnet, dass** die der flüssigen Paste zugesetzten Bakterien ausgewählt werden aus den zum Aktivieren der Produktion reduzierter Schwefelzusammensetzungen aus den Sulfaten des Mineralwassers und/oder zum Ausscheiden von Polymeren mit öligem Charakter fähigen Bakterien.

6. Verfahren nach Anspruch 5, **dadurch gekennzeichnet, dass** man Bakterien der Art Clostridium bifermentans verwendet.

7. Verfahren nach Anspruch 6, **dadurch gekennzeichnet, dass** die Bakterien in den Reaktor zu 10⁶ Bakterien pro Gramm Paste zugegeben werden.

8. Verfahren nach einem der Ansprüche 1 bis 7, **dadurch gekennzeichnet, dass** man Sulfate und/oder Sulfite und andere schweflige Derivate, zu 1 bis 3 g/kg Paste zusetzt.

9. Verfahren nach einem der vorstehenden Ansprüche, **dadurch gekennzeichnet, dass** der Schritt des Reifens aerob in einem offenen Reaktor durchgeführt wird, wenn man oxidische Pasten verwendet.

10. Verfahren nach einem der Ansprüche 1 bis 8, **dadurch gekennzeichnet, dass** der Schritt des Reifens anaerob in einem geschlossenen Reaktor durchgeführt wird, wenn man Pasten mit Redoxcharakter verwendet.

11. Verfahren nach Anspruch 10, **dadurch gekennzeichnet, dass** die Reifung bei einer Temperatur oberhalb von 35 bis 38 °C während wenigstens 30 Tagen durchgeführt wird, wenn man ein Inoculum (Impfung) von C. bifermentans verwendet.

12. Verfahren nach einem der Ansprüche 1 bis 11, **dadurch gekennzeichnet, dass** man einen Teil des Tränkwassers aus dem rückgewonnenen Schlammes evakuiert und dann den Schlamm einer thermischen Behandlung zum Zweck der Pasteurisierung und einer Konditionierung unterzieht.

13. Verfahren nach einem der vorstehenden Ansprüche, **dadurch gekennzeichnet, dass** es das Hinzufügen von Algen umfasst, die durch stetiges Berieseln eines ein Kulturnährmilieu bildenden Wassers unter natürlichem und/oder künstlichem Licht bei einer für die Entwicklung der Alge geeigneten Temperatur auf einem Kulturträger in geneigter Ebene erhalten werden.

14. Verfahren nach Anspruch 13, **dadurch gekennzeichnet, dass** die Algenstämme auf diesem Träger eingeimpft oder durch das verwendete Hyperthermalwasser, d. h. ein Mineralwasser, dessen Temperatur höher als etwa 45 °C ist, eingetragen werden.

15. Verfahren nach Anspruch 14, **dadurch gekennzeichnet, dass** das Wasser durch stickstoff- oder phosphorhaltige Nährstoffe ergänzt wird.

16. Verfahren nach Anspruch 14 oder 15, **dadurch gekennzeichnet, dass** man mit einem Mineralwasser bei einer Temperatur von 50 bis 56 °C die Berieselung mit einem Eintrag von Wasser von 0,1 bis 0,5 m³/h pro linearem Meter der Kultur auf einer zwischen 15 und 20 ° geneigten Ebene durchführt

17. Verfahren nach einem der Ansprüche 13 bis 16, **dadurch gekennzeichnet, dass** der Kulturträger beheizt wird, um das Absinken der Temperatur im unteren Teil der geneigten Ebene zu vermindern.

18. Verfahren nach Anspruch 5, **dadurch gekennzeichnet, dass** die zugesetzten Bakterien in einem Fermentator bei einer Temperatur von 38 °C unter Rühren und Steuerung des pH-Wertes bei einem Betrag von 6,0 ± 0,2 kultiviert werden.

19. Verfahren zum Zubereiten von Thermalschlämmen, **dadurch gekennzeichnet, dass** es in ein und derselben Zubereitungskette die in den Ansprüchen 1 bis 18 definierten Schritte umfasst.

20. Schlämme des Typs Thermalschlämme, enthaltend ein organo-mineralisches Material, das mit einem eine algen- und bakterienhaltige Biozönose enthaltenden Wasser sowie mit lonen mit therapeutischer Wirkung geimpft ist, **dadurch gekennzeichnet, dass** sie sich in Form einer homogenen Zusammensetzung von tonigem oder schlammigem Material darstellen und mindestens 10⁶ thermophile Bakterien des Schwefelzyklus pro Gramm enthalten.

21. Schlämme nach Anspruch 20, **dadurch gekennzeichnet, dass** sie reduzierte Schwefelderivate enthalten.

22. Schlämme nach einem der Ansprüche 20 oder 21, **dadurch gekennzeichnet, dass** ihnen Süßwasseralgen oder spezifische Thermalwasser-Algen zu 0,1 % des Trockengewichts zugesetzt sind.

23. Anwendung von Schlämmen nach einem der Ansprüche 20 bis 22 oder von nach dem Verfahren nach einem der Ansprüche 1 bis 19 erhaltenen Schlämmen im Bäderwesen.

24. Zubereitung zum paramedizinischen Gebrauch, **dadurch gekennzeichnet, dass** sie Schlämme nach einem der Ansprüche 20 bis 22 oder nach dem Verfahren nach einem der Ansprüche 1 bis 19 erhaltene Schlämme zusammen mit einem deren Anwendung erleichternden Vehikel des Typs Paraffin, Lanovaseline oder Methylcellulose enthält.

25. Kosmetische Zubereitung, **dadurch gekennzeichnet, dass** sie eine wirksame Menge von Schlämmen nach einem der Ansprüche 20 bis 22 oder von nach dem Verfahren nach einem der Ansprüche 1 bis 19 erhaltenen Schlämmen zusammen mit einem inerten, für die Anwendung in der Kosmetik geeigneten Vehikel enthält.

26. Kosmetische Zubereitung nach Anspruch 25, **dadurch gekennzeichnet, dass** sie thermophile Algen wie in Anspruch 3 definiert enthält.

27. Tiermedizinische Anwendung von Schlämmen nach einem der Ansprüche 20 bis 22 oder von nach dem Verfahren nach einem der Ansprüche 1 bis 19 erhaltenen Schlämmen.

## Claims

1. Process for the preparation of thermal muds, **characterised in that** it comprises the stages of
- mixing a fluid paste, produced by adding mineral water to an organo-mineral substrate constituted by an argillaceous or loamy material comprising an algal and bacterial biocoenosis, with sulphur cycle thermophilic bacteria, and if necessary fresh-water algae or specific algae from hyperthermal waters,
- maturation, under agitation, at an appropriate temperature, in order to activate the formation of metabolites by the bacteria, and the algae when they are used, and impregnation of the substrate,
- recovery of the mixture and its treatment.

2. Process according to claim 1, **characterised in that** Cyanophyceae or Cyanobacteria and/or Chlorophyceae and/or diatoms are used.

3. Process according to claim 2, **characterised in that** the algae are added to the fluid paste at the rate of 0.02 to 0.15 % as dry weight.

4. Process according to claim 3, **characterised in that** the algae are added at the rate of 0.05 to 0.10 % as dry weight.

5. Process according to one of claims 1 to 4, **characterised in that** the bacteria added to the fluid paste are chosen from the bacteria capable of activating the production of reduced compounds of sulphur from mineral water sulphates and/or secreting polymers of an unctuous nature.

6. Process according to claim 5, **characterised in that** bacteria of the genus Clostridium bifermentans are used.

7. Process according to claim 6, **characterised in that** the bacteria are added into the reaction vessel at the rate of 10⁶ bacteria/g of paste.

8. Process according to any one of claims 1 to 7, **characterised in that** sulphates and/or sulphites and other sulphur derivatives are added, at the rate of 1 to 3 g/kg of paste.

9. Process according to one of the previous claims, **characterised in that** the maturation stage is carried out in aerobiosis, in an open reaction vessel when oxidized pastes are used.

10. Process according to one of claims 1 to 8, **characterised in that** the maturation stage is carried out in anaerobiosis, in a closed reaction vessel, when pastes of a reducing nature are used.

11. Process according to claim 10, **characterised in that** maturation is carried out at a temperature greater than 35 to 38°C, for at least 30 days when a C.bifermentans inoculum is used.

12. Process according to one of claims 1 to 11, **characterised in that** part of the imbibition moisture is evacuated from the recovered mud, then the mud is subjected to a thermal treatment for pasteurisation purposes, and to conditioning operations.

13. Process according to one of the previous claims, **characterised in that** it comprises the addition of algae obtained by continuous streaming under natural and/or artificial light of a water constituting a nutritive culture medium, at a temperature suitable for the development of algae, on an inclined culture support.

14. Process according to claim 13, **characterised in that** the algae strains are sown onto this support or are carried by the hyperthermal water used, i.e. a mineral water, the temperature of which is greater than about 45°C.

15. Process according to claim 14, **characterised in that** the water is supplemented with nitrogenous or phosphorus nutrients.

16. Process according to claim 14 or 15, **characterised in that** with a mineral water at a temperature of 50 to 56°C, the streaming is effected with a rate of flow, per linear metre of culture, of 0.1 to 0.5 m³/h on an inclined plane of 15 to 20°.

17. Process according to one of claims 13 to 16, **characterised in that** the culture support is heated in order to reduce the drop in temperature in the lower part of the inclined plane.

18. Process according to claim 5, **characterised in that** the added bacteria are cultured in a fermentation chamber, at a temperature of 38°C, accompanied by stirring, with pH controlled at a value of 6.0 ±0.2.

19. Process for the preparation of thermal muds, **characterised in that** it comprises in a single preparation line the stages described in claims 1 to 18.

20. Muds of the thermal mud type, containing an organo-mineral substrate imbibed from a mineral water comprising an algal and bacterial biocoenosis as well as ions with a therapeutic effect, **characterised in that** they are present in the form of a homogeneous composition of argillaceous or loamy material, and contain at least 10⁶ sulphur cycle thermophilic bacteria/g.

21. Muds according to claim 20, **characterised in that** they contain reduced sulphur derivatives.

22. Muds according to any one of claims 20 or 21, **characterised in that** fresh water algae or specific algae from thermal waters are added at the rate of 0.1% in dry weight.

23. Use of the muds according to any one of claims 20 to 22, or those obtained by the process according to any one of claims 1 to 19 in spas or hydrotherapy.

24. Preparation for paramedical use, **characterised in that** it contains muds according to any one of claims 20 to 22, or those obtained by the process according to one of claims 1 to 19, combined with a vehicle facilitating their use, of paraffin, lanovaseline or methylcellulose type.

25. Cosmetological preparations, **characterised in that** it contains an effective quantity of muds according to any one of claims 20 to 22, or those obtained by the process according to one of claims 1 to 19, combined with an inert vehicle suitable for use in cosmetology.

26. Cosmetological preparations according to claim 25, **characterised in that** it contains thermophilic algae such as those described in claim 3.

27. Veterinary use of the muds according to any one of claims 20 to 22, or those obtained by the process according to one of claims 1 to 19.
